(19) Europäisches Patentamt / European Patent Office / Office européen des brevets

(11) **EP 3 793 424 B1**

(12) **FASCICULE DE BREVET EUROPEEN**

(45) Date de publication et mention de la délivrance du brevet:
**19.03.2025 Bulletin 2025/12**

(21) Numéro de dépôt: **18740628.5**

(22) Date de dépôt: **07.06.2018**

(51) Classification Internationale des Brevets (IPC):
***A61B 3/028*** *(2006.01)*

(52) Classification Coopérative des Brevets (CPC):
**A61B 3/0285**

(86) Numéro de dépôt international:
**PCT/FR2018/051326**

(87) Numéro de publication internationale:
**WO 2019/220023 (21.11.2019 Gazette 2019/47)**

(54) **DISPOSITIF, PROCÉDÉ ET CABINE DE DÉTERMINATION AUTOMATIQUE DE LA RÉFRACTION OCULAIRE SUBJECTIVE D'UN PATIENT**

VORRICHTUNG, VERFAHREN UND KABINE ZUR AUTOMATISCHEN BESTIMMUNG DER SUBJEKTIVEN AUGENBRECHUNG EINES PATIENTEN

DEVICE, METHOD AND BOOTH FOR AUTOMATIC DETERMINATION OF THE SUBJECTIVE OCULAR REFRACTION OF A PATIENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.05.2018 FR 1854106**

(43) Date de publication de la demande:
**24.03.2021 Bulletin 2021/12**

(73) Titulaires:
• **Costantini, Florent**
  **75016 Paris (FR)**
• **Costantini, Mathieu**
  **20090 Ajaccio (FR)**

(72) Inventeurs:
• **Costantini, Florent**
  **75016 Paris (FR)**
• **Costantini, Mathieu**
  **20090 Ajaccio (FR)**

(74) Mandataire: **Gevers & Orès**
  **Immeuble le Palatin 2**
  **3 Cours du Triangle**
  **CS 80165**
  **92939 Paris La Défense Cedex (FR)**

(56) Documents cités:
US-A1- 2009 153 796     US-A1- 2015 070 650
US-A1- 2015 150 446     US-A1- 2016 310 000
US-A1- 2017 329 154

## Description

### DOMAINE TECHNIQUE

La présente invention concerne un dispositif et un procédé de détermination automatique de la réfraction oculaire subjective d'un patient, ainsi qu'une cabine pourvue d'un tel dispositif.

La réfraction oculaire est le changement de direction d'un rayon lumineux qui traverse, dans l'œil, des milieux différents. Ce rayon finit par converger sur la rétine, c'est-à-dire sur la couche de cellules qui tapissent le fond du globe oculaire et qui est destinée à transformer l'énergie lumineuse en influx nerveux décodé ensuite par le cerveau sous la forme d'images.

La réfraction oculaire permet de déterminer des anomalies de la vue (amétropies) d'une personne (nommé « patient » ci-après). Les amétropies d'un œil sont définies par la position des rayons lumineux entrant dans l'œil, qui sont déviés par rapport à la rétine. Selon la position de ces rayons, l'œil est considéré comme myope, normal ou hypermétrope. De plus, lorsque les rayons lumineux décrivent une surface sur la rétine en lieu et place d'un point, l'œil est astigmate.

### ÉTAT DE LA TECHNIQUE

Pour déterminer la réfraction oculaire d'un patient, on utilise généralement un réfractomètre automatique (ou « auto-réfractomètre ») qui permet de mesurer une réfraction théorique dite réfraction objective, qui définit une correction objective pour chaque œil du patient.

Toutefois, l'état des yeux d'un patient étant susceptible de varier au cours du temps, les résultats fournis par un réfractomètre automatique sont variables au cours du temps. Des phénomènes musculaires et neurosensoriels comme l'accommodation peuvent également faire varier ces résultats. Aussi, lors d'un examen ou consultation ophtalmologique, notamment en vue de prescrire de verres correcteurs adaptés au patient, l'ophtalmologiste (ou un autre professionnel de la vision tel qu'un orthoptiste, un optométriste ou un opticien) détermine une réfraction oculaire dite subjective du patient, en requérant la participation du patient.

Pour ce faire, le professionnel de la vision (ophtalmologiste, orthoptiste, optométriste ou opticien par exemple) utilise généralement un réfracteur automatique. De façon usuelle, un réfracteur automatique comprend un dispositif oculaire à deux oculaires destiné à être placé devant les yeux du patient, une unité de gestion configurée pour prélever des verres correcteurs d'une réserve et les positionner dans les oculaires, ainsi qu'une interface de commande qui est utilisée par le professionnel de la vision pour commander l'unité de gestion. Au cours de cet examen, différentes combinaisons de verres correcteurs sont placés dans les oculaires, et le patient vérifie, en regardant une cible à travers le dispositif oculaire, si le ou les verres présentés permettent une vision nette de la cible.

Le professionnel de la vision doit, généralement, mettre en œuvre un grand nombre de tests successifs avant de pouvoir déterminer une correction visuelle confortable adaptée au patient. Un tel examen présente donc une durée importante.

Par le document FR 3 050 922, on connaît un procédé permettant de réduire la durée d'un examen de la réfraction oculaire subjective d'un patient. Ce procédé qui a pour objet de commander un réfracteur automatique est caractérisé par une étape de détermination d'un profil accommodatif indiquant une capacité de l'œil à accommoder de façon excessive ou non, et une étape de génération d'au moins une commande de sélection identifiant des verres correcteurs à positionner successivement dans le dispositif oculaire en vue de soumettre l'œil à plusieurs tests de réfraction successifs, la commande de sélection dépendant du profil accommodatif déterminé. Ce procédé permet de déterminer automatiquement un ensemble de tests en identifiant des verres correcteurs à positionner dans le dispositif oculaire, ce qui permet de réduire la durée de l'examen.

Au cours de l'examen, les tests oculaires sont, toutefois, toujours réalisés de façon usuelle. En particulier :

- le patient est guidé par un opérateur tel qu'un professionnel de la vision qui contrôle l'exécution du ou des tests (déterminés automatiquement) ; et
- l'opérateur entre les réponses fournies par le patient dans le système au cours des tests.

Ce procédé, bien que partiellement automatisé, nécessite donc toujours l'intervention d'un opérateur, généralement un professionnel de la vision, ce qui présente des inconvénients notamment en termes de coût et de disponibilité.

US 2009/153796 décrit un système optométrique-ophtalmique comportant:

- Unité montable sur la tête (14) avec module oculaire proche des yeux (20)
- Ensembles de lentilles avec positionnement automatique dans le chemin optique
- Unité d'entrée de données patient (52) pour saisie d'informations
- Unité d'affichage (54) montrant notamment des optotypes E
- Interface joystick (56) pour recueillir les réponses du patient
- Contrôle automatique par unité centrale
- Modification automatique de la puissance réfractive selon réponses patient (alinéa 226).

US 2015/070650 révèle un système d'autophoroptère/auto-réfractomètre (alinéa 36) comportant:

- Réfracteur automatique avec contrôle à distance (alinéa 39)
- Auto-réfractomètre intégré (alinéa 36)
- Frontofocomètre (auto-lensometer, alinéa 36)
- Interface de saisie des données patient (alinéas 37, 64-65, 72)
- Système de génération d'instructions auditives pour le patient (alinéas 41, 51)
- Interface joystick pour les réponses du patient (alinéa 57)
- Cabine d'examen dédiée (alinéa 53)
- Génération automatique de tests de réfraction basés sur les réponses (alinéa 41)
- Production d'ordonnances automatisée (alinéa 40).

## EXPOSÉ DE L'INVENTION

La présente invention prévoit un dispositif de détermination automatique au moins de la réfraction oculaire subjective d'un patient, qui permet de remédier à ces inconvénients. Elle concerne un dispositif comportant les caractéristiques techniques des revendications, notamment :

- un réfracteur automatique comprenant un dispositif oculaire (à un ou deux oculaires) et une unité de gestion configurée pour prélever des verres correcteurs d'une réserve et les positionner dans le dispositif oculaire ;
- une unité centrale configurée pour au moins commander l'unité de gestion du réfracteur automatique afin de positionner des verres correcteurs dans le dispositif oculaire ; et
- une unité de saisie de données permettant d'entrer dans l'unité centrale au moins des données relatives au patient.

Selon l'invention :

- ledit dispositif comporte de plus :

  • un système de génération d'instructions commandé par l'unité centrale et configuré pour générer automatiquement des instructions destinées au patient au moins lors d'un test oculaire ; et

  • un système d'interface entre le patient et l'unité centrale, qui est configuré pour détecter, directement, des indications générées sous forme gestuelle, de préférence manuelle, par le patient au moins lors d'un test oculaire et pour transmettre directement ces indications à l'unité centrale ; et

- l'unité centrale est configurée également pour mettre en œuvre automatiquement au moins un test oculaire, en commandant l'unité de gestion et en recevant les indications générées par le patient par l'intermédiaire du système d'interface, et pour déterminer automatiquement la réfraction oculaire subjective à partir au moins desdites indications reçues.

De préférence, l'unité centrale est configurée pour générer une ordonnance comprenant une prescription de lunettes pour le patient, adaptée à ladite réfraction oculaire subjective ainsi déterminée.

Dans le cadre de la présente invention, on entend, par détection et/ou transmission « directes » d'indications gestuelles (ou indications générées de manière gestuelle), la détection sans intervention d'un tiers (tel qu'un opérateur notamment un professionnel de la vision) de ces indications gestuelles et la transmission de ces indications gestuelles à l'unité centrale du dispositif, également sans intervention d'un tiers.

Ainsi, grâce simultanément à la génération automatique d'instructions au patient (via le système de génération d'instructions) en vue notamment de la mise en œuvre d'un ou de plusieurs tests oculaires et à la détection directe et à la prise en compte directe des réponses du patient (exprimées sous forme de gestes) lors de ce ou ces tests oculaires (via le système d'interface), ledit dispositif ne nécessite pas l'intervention d'un opérateur tel qu'un professionnel de la vision, pour mettre en œuvre le ou les tests oculaires nécessaires à l'unité centrale du dispositif pour déterminer la réfraction oculaire subjective du patient. Ledit dispositif permet ainsi, par sa seule collaboration avec le patient, sans intervention d'un tiers (tel qu'un opérateur), de déterminer automatiquement la réfraction oculaire subjective du patient et de générer automatiquement une ordonnance le cas échéant, ce qui apporte des avantages notamment en termes de coût et de disponibilité et permet de remédier aux inconvénients précités.

Avantageusement, ledit dispositif comporte, de plus, au moins l'un des éléments suivants :

- un élément d'enregistrement configuré pour enregistrer l'ordonnance ; et
- un élément d'impression configuré pour imprimer l'ordonnance.

Par ailleurs, dans un mode de réalisation particulier, le dispositif comporte, de plus, un élément de transmission configuré pour transmettre l'ordonnance à distance à un utilisateur.

En outre, dans un mode de réalisation préféré, le système de génération d'instructions comporte une unité d'émission vocale.

Dans le cadre de la présente invention :

- le système d'interface est un système qui permet de détecter automatiquement des gestes du patient, qu'il réalise pour fournir (ou générer) lesdites indications (gestuelles), qui représentent ses commentaires (ou réponses) relatifs aux questions posées lors du ou des tests oculaires ;

- les gestes du patient correspondent à des mouvements d'au moins une partie de son corps. De préférence, ces mouvements concernent des déplacements ou des actions réalisés par l'une ou les deux mains du patient. Toutefois, l'utilisation d'autres parties du corps, telles que par exemple les pieds, peut être envisagée dans le cadre de la présente invention.

Dans un premier mode de réalisation, le système d'interface comporte au moins un organe d'actionnement manuel. Cet organe d'actionnement manuel est configuré pour pouvoir être actionné manuellement par le patient. Il peut notamment s'agir d'un ou de plusieurs éléments d'actionnement tels que des boutons. Il peut également s'agir d'une manette. Dans ce premier mode de réalisation, les indications gestuelles représentent donc des actionnements manuels de l'organe d'actionnement manuel.

En outre, dans un second mode de réalisation, le système d'interface comporte un système de détection automatique de gestes du patient. Dans ce second mode de réalisation, les indications (gestuelles) représentent des gestes, notamment des mouvements en particulier d'une ou des deux mains, qui sont détectés automatiquement et directement par ledit système de détection automatique de gestes (et transmis à l'unité centrale).

Ledit dispositif comporte, de plus :

- un réfractomètre automatique configuré pour mesurer la réfraction oculaire objective du patient et pour la transmettre à l'unité centrale, la réfraction oculaire objective mesurée étant utilisée par l'unité centrale pour mettre en œuvre au moins l'une des deux actions suivantes : générer ledit au moins un test oculaire, déterminer ladite réfraction oculaire subjective ; et

- un frontofocomètre configuré pour mesurer les corrections de verres de lunettes du patient et pour transmettre les corrections mesurées à l'unité centrale, les corrections mesurées étant utilisées par l'unité centrale pour mettre en œuvre au moins l'une des deux actions suivantes : générer ledit au moins un test oculaire, déterminer ladite réfraction oculaire subjective ; et

- un système de commande vocale permettant au patient de fournir au dispositif des indications vocales.

Dans le cadre de la présente invention, les différents éléments (et notamment le réfracteur automatique, l'unité centrale, l'unité de saisie de données, le système de génération d'instructions et le système d'interface, ainsi que le cas échéant le réfractomètre automatique et le frontofocomètre) du dispositif peuvent être reliés ensemble de différentes manières, pour communiquer et transmettre des données et/ou des commandes notamment.

Ainsi, avantageusement :

- dans un premier mode de réalisation, ledit dispositif comporte un réseau local configuré pour permettre des communications entre au moins certains desdits éléments dudit dispositif ;
- dans un deuxième mode de réalisation, au moins certains desdits éléments dudit dispositif communiquent entre eux par l'intermédiaire de liaisons série ; et
- dans un troisième mode de réalisation, au moins certains desdits éléments dudit dispositif communiquent entre eux par l'intermédiaire d'une communication sans fil (ou « wifi »).

La présente invention concerne également une cabine de détermination automatique de la réfraction oculaire subjective d'un patient (et de préférence, de génération d'une ordonnance).

Selon l'invention, ladite cabine est au moins partiellement fermée, et elle comporte au moins un siège pour un patient et un dispositif de détermination automatique de la réfraction oculaire subjective d'un patient, tel que celui spécifié ci-dessus.

Avantageusement, les différents éléments (dudit dispositif de détermination automatique de la réfraction oculaire subjective) sont agencés dans la cabine de manière à être accessibles, si nécessaire, à partir du siège du patient.

La présente invention concerne, en outre, un procédé de détermination automatique de la réfraction oculaire subjective d'un patient, à l'aide d'un dispositif tel que celui précité, qui comporte au moins :

- un réfracteur automatique comprenant un dispositif oculaire et une unité de gestion configurée pour prélever des verres correcteurs d'une réserve et les positionner dans le dispositif oculaire ;
- une unité centrale ;
- une unité de saisie de données ;
- un système de génération d'instructions ; et
- un système d'interface entre le patient et l'unité centrale.

Selon l'invention, ledit procédé comporte :

- une étape de génération, mise en œuvre par l'unité centrale, et consistant à générer automatiquement au moins un test oculaire, à l'aide au moins de données entrées, relatives au patient ;
- une étape de test consistant à mettre en œuvre au moins un test oculaire généré à l'étape de génération, l'étape de test comprenant au moins :

  • une sous-étape de génération d'instructions pour le patient, mise en œuvre par le système de génération d'instructions ;

- une sous-étape de commande de l'unité de gestion du réfracteur automatique pour prélever des verres correcteurs de la réserve et les positionner dans le dispositif oculaire ;
- une sous-étape de détection directe d'indications générées sous forme gestuelle par le patient au cours du test oculaire, en regardant des affichages à travers le dispositif oculaire, et de transmission directe de ces indications à l'unité centrale, mise en œuvre par le système d'interface ; et

- une étape de traitement, mise en œuvre par l'unité centrale, et consistant à déterminer automatiquement la réfraction oculaire subjective du patient, à partir au moins desdites indications reçues, ainsi qu'une ordonnance.

Avantageusement, ledit procédé comporte, de plus, au moins une étape d'entrée de données, qui est mise en œuvre antérieurement à l'étape de génération.

De préférence, les étapes de génération et de test utilisent un test de brouillard.

De plus, dans un mode de réalisation préféré, l'étape de test consiste à afficher des lettres E avec des branches présentant des orientations différentes, qui peuvent être facilement et simplement reconnues, notamment par des populations d'individus illettrés.

## BRÈVE DESCRIPTION DES FIGURES

L'invention sera mieux comprise, et d'autres buts, détails, caractéristiques et avantages de celle-ci apparaîtront plus clairement au cours de la description explicative détaillée qui va suivre, de modes de réalisation de l'invention donnés à titre d'exemple purement illustratif et non limitatif, en référence aux dessins schématiques annexés. Sur ces dessins :

- la figure 1 est un schéma synoptique d'un mode de réalisation particulier d'un dispositif selon l'invention de détermination de la réfraction oculaire subjective d'un patient ;
- la figure 2 montre schématiquement un premier mode de communication entre différents éléments du dispositif, via un réseau local ;
- la figure 3 montre schématiquement un second mode de communication entre différents éléments du dispositif, via des liaisons séries ;
- la figure 4 est une vue schématique, en perspective, d'une cabine de détermination de la réfraction oculaire subjective d'un patient ;
- les figures 5 et 6 sont des vues schématiques en perspective, en regardant respectivement de la droite et de la gauche, de la cabine de la figure 4 ; et
- la figure 7 montre schématiquement les étapes principales d'un mode de réalisation particulier d'un procédé de détermination automatique de la réfraction oculaire subjective d'un patient.

## DESCRIPTION DÉTAILLÉE

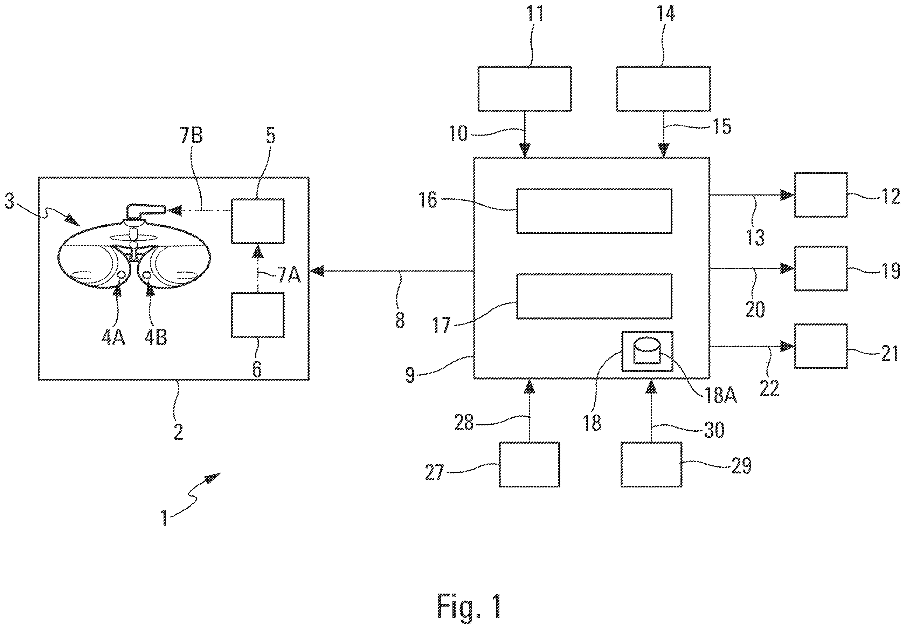

Le dispositif 1, représenté schématiquement sur la figure 1 et permettant d'illustrer la présente invention, est un dispositif de détermination automatique au moins de la réfraction oculaire subjective d'un patient.

De façon usuelle, ce dispositif 1 comporte :

- un réfracteur automatique 2 comprenant un dispositif oculaire 3 pourvu d'un ou de préférence de deux oculaires 4A et 4B et d'une unité de gestion 5 configurée pour prélever des verres correcteurs d'une réserve 6 par exemple à l'aide d'un bras articulé (non représenté) et les positionner dans les oculaires 4A et 4B, comme illustré schématiquement par des traits mixtes 7A et 7B sur la figure 1 ;
- une unité centrale 9 configurée pour au moins commander l'unité de gestion 5 du réfracteur automatique 2, comme illustré par une liaison 8, afin de positionner des verres dans le ou les oculaires 4A et 4B du dispositif oculaire 3 ; et
- une unité de saisie de données 11 permettant d'entrer dans l'unité centrale 9 au moins des données relatives au patient, comme illustré par une liaison 10.

L'unité de saisie de données 11 peut comporter tout type d'éléments ou de moyens usuels permettant d'entrer des données, tels que par exemple un clavier associé à un écran ou un écran tactile.

Selon l'invention, ledit dispositif 1 comporte de plus :

- un système de génération d'instructions 12 commandé par l'unité centrale 9, comme illustré par une liaison 13, et configuré pour générer des instructions au patient au moins lors d'un test oculaire ; et
- un système d'interface 14 créant une interface entre le patient et l'unité centrale 9.

Selon l'invention, le système d'interface 14 est configuré pour détecter, directement, des indications générées par le patient sous forme gestuelle, et de préférence sous forme manuelle, au moins lors d'un test oculaire, et pour transmettre directement ces indications (gestuelles) détectées à l'unité centrale 9 via une liaison 15.

De plus, selon l'invention, l'unité centrale 9 comporte un élément de commande 16 qui est configuré :

- pour mettre en œuvre automatiquement au moins un test oculaire, en commandant l'unité de gestion 5, en guidant le patient par l'intermédiaire du système de génération d'instructions 12 et en recevant les indications générées par le patient par l'intermédiaire du système d'interface 14 ; et

- pour déterminer automatiquement la réfraction oculaire subjective à partir au moins desdites indications reçues.

L'unité centrale 9 comporte également un élément de traitement 17 configuré pour générer une ordonnance. Cette ordonnance comprend notamment une prescription de verres correcteurs de lunettes pour le patient, dont la correction est adaptée à la réfraction oculaire subjective déterminée par l'élément de commande 16.

Ledit dispositif 1 comporte, de plus, un élément d'enregistrement 18 qui est configuré pour enregistrer au moins l'ordonnance (et éventuellement la réfraction oculaire objective) générée par l'élément de traitement 17, sur un moyen de stockage 18A, interne ou externe (à l'unité centrale 9), de tout type usuel, par exemple une clef USB.

En complément et/ou en variante, le dispositif 1 comporte un élément d'impression (ou imprimante) 19. Cet élément d'impression 19 est configuré pour imprimer sur du papier l'ordonnance générée par l'élément de traitement 17 (ou simplement la réfraction oculaire subjective) et reçue par l'intermédiaire d'une liaison 20. Ainsi, le patient disposera immédiatement après l'examen d'une ordonnance sous forme d'un document papier, lui permettant d'obtenir des lunettes adaptées à sa vue.

Par ailleurs, dans un mode de réalisation particulier, le dispositif 1 comporte, en complément et/ou en variante, un élément de transmission 21. Cet élément de transmission 21 est configuré pour transmettre (notamment à distance via internet) l'ordonnance, générée par l'élément de traitement 17 (ou simplement la réfraction oculaire subjective) et reçue par l'intermédiaire d'une liaison 22, à un utilisateur, par exemple à un professionnel de la santé.

Ainsi, le dispositif 1 est en mesure de réaliser une télétransmission de l'ordonnance et/ou de la réfraction oculaire subjective.

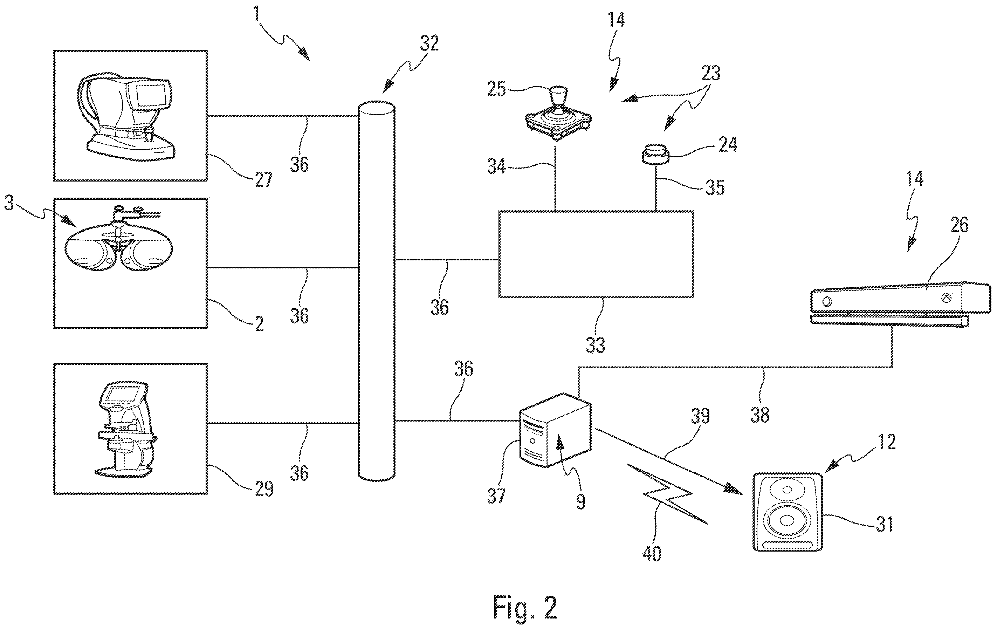

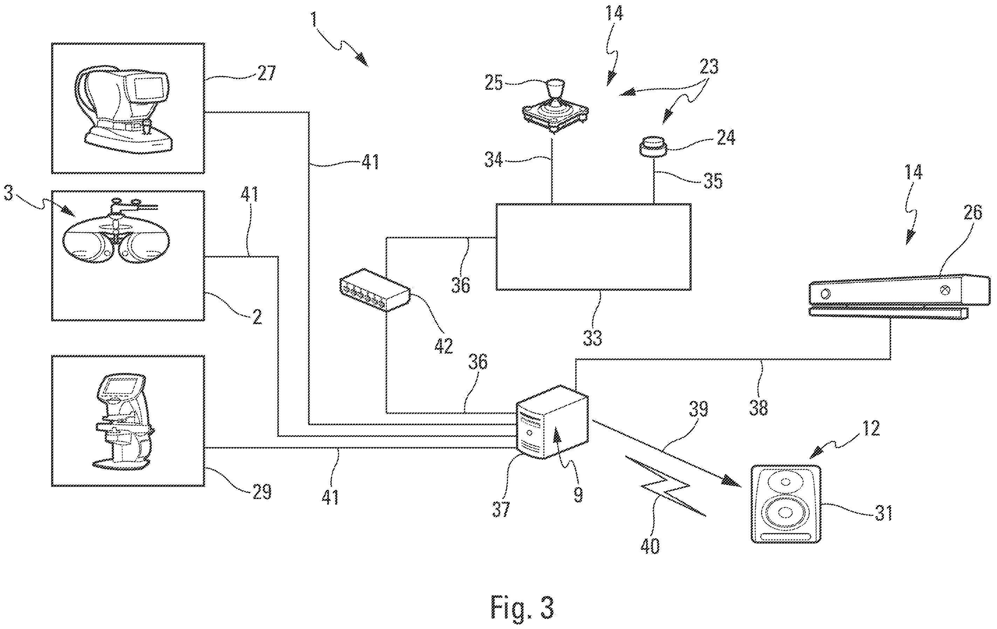

Dans un mode de réalisation préféré, le système de génération d'instructions 12 comporte une unité d'émission vocale usuelle, comprenant un haut-parleur 31 (figures 2 et 3), qui est configurée pour générer les instructions sous forme de signaux vocaux, c'est-à-dire via une voix synthétisée. Ces instructions ont notamment pour but de guider le patient, en particulier lors de tests oculaires, comme précisé ci-dessous. En variante, il est également envisageable que le système de génération d'instructions 12 génère les instructions sous forme visuelle.

Dans le cadre de la présente invention :

- le système d'interface 14 est un système qui permet de détecter automatiquement des gestes du patient, à savoir les gestes que le patient réalise pour fournir (ou générer) lesdites indications (gestuelles), qui représentent ses commentaires (ou réponses) relatifs notamment à des questions posées lors d'un test oculaire ;
- les gestes du patient correspondent à des mouvements d'une partie du corps dudit patient. De préférence, ces mouvements concernent des déplacements ou des actions réalisés par l'une des mains ou les deux mains du patient. Toutefois, des mouvements d'autres parties du corps du patient, peuvent être envisagés dans le cadre de la présente invention, tels que notamment les mouvements de ses pieds qui agissent, par exemple, sur des pédales ou d'autres éléments mécaniques (faisant partie dans ce cas du système d'interface 14).

Dans un premier mode de réalisation, le système d'interface 14 comporte au moins un organe d'actionnement manuel 23. Cet organe d'actionnement manuel 23 est configuré pour pouvoir être actionné manuellement par le patient. Il peut s'agir en particulier d'un ou de plusieurs éléments d'actionnement tels que des boutons, par exemple des boutons-poussoirs ou des boutons rotatifs, comme représenté par exemple sur les figures 2 et 3 avec le bouton 24. A titre d'illustration, l'organe d'actionnement manuel 23 peut comporter deux boutons, dont l'un indique par exemple la droite et l'autre la gauche. L'organe d'actionnement manuel 23 peut également être une manette 25 usuelle, comme représenté sur les figures 2 et 3. Une telle manette 25, de type « joystick » notamment, peut par exemple être déplacée dans deux directions différentes, une direction avant/arrière et une direction haut/bas. Dans ce premier mode de réalisation, les indications gestuelles représentent ainsi des actionnements manuels de l'organe d'actionnement manuel 23 par le patient.

En outre, dans un second mode de réalisation, le système d'interface 14 comporte un système de détection 26 (figures 2 et 3) de type usuel, qui est configuré pour détecter automatiquement (à distance, c'est-à-dire sans contact) les gestes du patient. Dans ce second mode de réalisation, les indications gestuelles représentent des gestes, notamment des mouvements d'une ou des deux mains du patient, qui sont détectés automatiquement et directement par le système de détection 26 sans intervention d'un tiers et sans contact du patient.

Par ailleurs, dans un mode de réalisation particulier, le dispositif 1 comporte un système de commande vocale (non représenté) qui permet au patient de fournir audit dispositif 1 (et en particulier à l'unité centrale 9) des indications sous forme vocale, notamment lors d'un test oculaire.

Ledit dispositif 1 comporte, de plus, comme représenté sur la figure 1, un réfractomètre automatique 27 usuel configuré pour mesurer la réfraction oculaire objective du patient. Pour effectuer cet examen, le patient doit poser son front et son menton contre et sur des supports appropriés. Le réfractomètre automatique 27 mesure alors automatiquement la réfraction oculaire objective des yeux du patient. Le réfractomètre automatique 27 est configuré pour transmettre automatiquement à l'unité centrale 9, par l'intermédiaire d'une liaison 28, la réfraction oculaire objective ainsi mesurée. Cette réfraction oculaire objective mesurée est utilisée par l'unité centrale 9 pour générer automatiquement le ou les tests oculaires et/ou pour déterminer automatiquement ladite réfraction oculaire subjective.

Ledit dispositif 1 comporte, de plus, comme représenté sur la figure 1, un frontofocomètre 29 usuel configuré pour mesurer les corrections de verres de lunettes du patient. Dans un mode de réalisation préféré, le frontofocomètre 29 est automatisé. Ce frontofocomètre 29 est configuré pour transmettre à l'unité centrale 9, par l'intermédiaire d'une liaison 30, les corrections mesurées. Ces corrections mesurées sont utilisées par l'unité centrale 9 pour générer automatiquement le ou les tests oculaires et/ou pour déterminer automatiquement ladite réfraction oculaire subjective.

Dans le cadre de la présente invention, les différents éléments (et notamment le réfracteur automatique 2, l'unité centrale 9, l'unité de saisie de données 11, le système de génération d'instructions 12 et le système d'interface 14, ainsi que le cas échéant le réfractomètre automatique 27 et le frontofocomètre 29) du dispositif 1 peuvent être reliés ensemble de différentes manières, pour communiquer et transmettre des données et/ou des commandes notamment.

Dans un premier mode de réalisation, représenté sur la figure 2, le dispositif 1 comporte un réseau informatique (local) 32 de type LAN (pour « Local Area Network » en anglais) qui est configuré pour permettre des communications entre au moins certains des éléments précités du dispositif 1.

Sur l'exemple des figures 2 et 3, le système d'interface 14 comprend un nano-ordinateur 33 équipé d'un processeur ARM, par exemple de type Raspberry PI. Le bouton 24 et la manette 25 sont reliés, chacun, au nano-ordinateur 33, par l'intermédiaire d'une liaison USB ou d'un bus IO Raspberry PI, c'est-à-dire d'un ensemble de connectiques électroniques entre les différentes unités (entrées ou sorties) de la carte électronique Raspberry PI, comme illustré par des liaisons 34 et 35.

Dans l'exemple représenté sur la figure 2, le réfracteur automatique 2, l'unité centrale 9, le nano-ordinateur 33, le réfractomètre automatique 27 et le frontofocomètre 29 sont liés (ou connectés) au réseau local 32 via des connecteurs 36 usuels, par exemple de type RJ45.

En outre, dans les deux modes de réalisation des figures 2 et 3, l'unité centrale 9 qui est, par exemple, intégrée dans un ordinateur 37 est liée au système de détection 26 par l'intermédiaire d'une liaison 38, par exemple de type USB. De plus, l'unité centrale 9 est liée au système de génération d'instructions 12, par l'intermédiaire d'une liaison filaire 39 (prise « jack » ou « RCA ») ou d'une liaison 40 de type « bluetooth ».

En outre, dans un deuxième mode de réalisation, représenté sur la figure 3, au moins certains des éléments du dispositif 1 communiquent entre eux par l'intermédiaire de liaisons série 41. A titre d'illustration, dans l'exemple représenté sur cette figure 3, le réfracteur automatique 2, le réfractomètre automatique 27 et le frontofocomètre 29 sont liés (ou connectés) à l'unité centrale 9, par l'intermédiaire de telles liaisons 41, par exemple des liaisons RS232 de type UART (pour « Universal Asynchronous Receiver Transmitter » en anglais).

Dans ce deuxième mode de réalisation, le dispositif 1 comporte également un dispositif 42 de type « HUB » ou « Switch », qui relie par l'intermédiaire de connecteurs 36, par exemple de type RJ45, le nano-ordinateur 33 du système d'interface 14 à l'unité centrale 9.

En outre, dans un troisième mode de réalisation (non représenté), au moins certains des éléments du dispositif 1 communiquent entre eux par l'intermédiaire d'une communication sans fil (ou « wifi »).

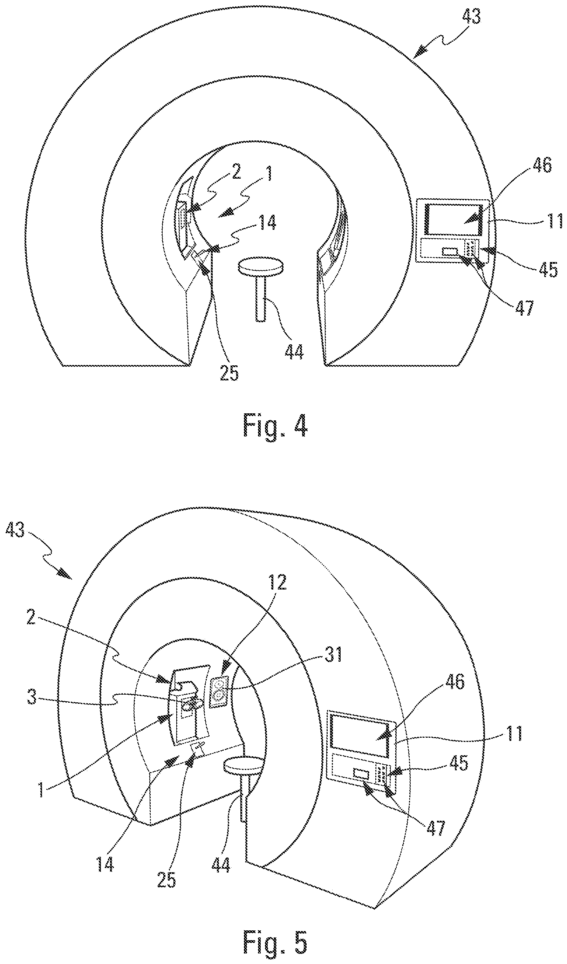

Dans une application préférée, représentée sur les figures 4 à 6, le dispositif 1 de détermination automatique de la réfraction oculaire subjective d'un patient, tel que décrit ci-dessus, est intégré dans une cabine 43 (de détermination automatique de la réfraction oculaire subjective). Cette cabine 43 est au moins partiellement fermée et elle comporte, en plus dudit dispositif 1, un siège 44 pour un patient. Ce siège 44 est réglable, notamment en hauteur et éventuellement dans le plan horizontal.

Les différents éléments dudit dispositif 1 et notamment le réfracteur automatique 2, le système d'interface 14, le réfractomètre automatique 27 et le frontofocomètre 29 sont agencés de manière à être accessibles, à partir dudit siège 44 du patient.

Le refractomètre automatique 27 et le frontofocomètre 29 peuvent être optionnels dans un mode de réalisation simplifié.

A titre d'illustration, l'unité de saisie de données 11, par exemple un clavier 45 associé à un écran 46, peut être agencée à l'entrée de la cabine 43, comme représenté sur l'exemple des figures 4 à 6. L'unité de saisie de données 11 peut également être agencée à l'intérieur de la cabine 43. L'unité de données 11 peut également comprendre un terminal de paiement 47, pour un paiement, par exemple par carte bancaire.

Dans l'exemple de réalisation représenté, la cabine 43 comporte à l'intérieur :

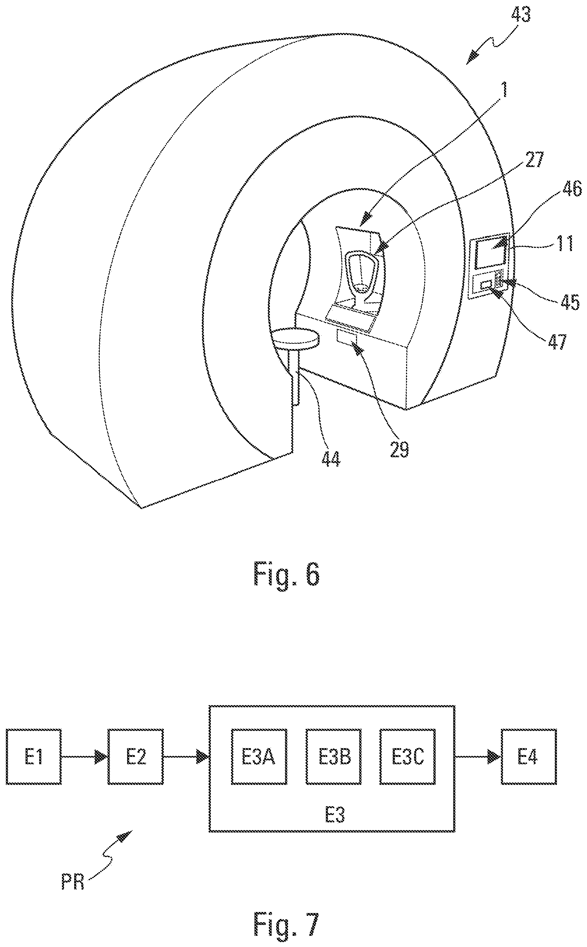

- sur l'un des côtés, comme montré sur la figure 5, le réfracteur automatique 2, le système d'interface 14, et le système de génération d'instructions 12 ; et
- sur l'autre côté, comme représenté sur la figure 6, le réfractomètre automatique 27 et le frontofocomètre 29.

On décrit ci-dessous, en référence à la figure 7 en lien avec la figure 1, un procédé PR de détermination automatique de la réfraction oculaire subjective d'un patient, mis en œuvre à l'aide d'un dispositif 1 tel que celui décrit ci-dessus.

Ledit procédé PR comporte :

- une étape d'entrée de données E1 consistant à entrer des données relatives au patient dans le dispositif 1 (et plus particulièrement dans l'unité centrale 9) ;
- une étape de génération E2, mise en œuvre par l'unité centrale 9, et consistant à générer automatiquement au moins un test oculaire, à l'aide au moins de données entrées, relatives au patient ;
- une étape de test E3 pour mettre en œuvre au moins un test oculaire généré à l'étape de génération E2. L'étape de test E3 comprend au moins :

  - une sous-étape E3A de génération d'instructions pour le patient, mise en œuvre par le système de génération d'instructions 12 ;
  - une sous-étape E3B de commande de l'unité de gestion 5 du réfracteur automatique 2 pour prélever des verres correcteurs dans la réserve 6 et les positionner dans les oculaires 4A et 4B du dispositif oculaire 3 ;
  - une sous-étape E3C de détection directe d'indications gestuelles générées par le patient au cours du test oculaire, au cours duquel le patient regarde des affichages commandés par l'unité centrale 9 au travers du dispositif oculaire 3 pourvu de verres correcteurs particuliers et il génère des indications en fonction de ce qu'il voit, la sous-étape E3C (mise en œuvre par le système d'interface 14) comprenant également une transmission directe de ces indications à l'unité centrale 9 ; et

- une étape de traitement E4, mise en œuvre par l'unité centrale 9, et consistant à déterminer automatiquement à partir notamment desdites indications reçues, la réfraction oculaire subjective du patient et de préférence également une ordonnance. L'étape de traitement E4 détermine, de préférence, la réfraction oculaire subjective du patient, à l'aide notamment de l'âge du patient, de sa réfraction oculaire objective RO mesurée par le réfractomètre automatique 27, de sa correction optique portée mesurée à l'aide du frontofocomètre 29 et de ses réponses recueillies par l'unité centrale 9 via le système d'interface 14 lors des tests oculaires.

On précise ci-dessous, plus en détail, les étapes E1 à E4 du procédé PR.

A l'étape d'entrée de données E1, le patient entre (à l'aide de l'unité de saisie de données 11) des données le concernant, et notamment son âge, qui seront utilisées par l'unité centrale 9 pour déterminer la réfraction oculaire subjective.

Dans un mode de réalisation particulier, on peut envisager :

- que le patient réalise un paiement préalable à la consultation, par exemple à l'aide du terminal de paiement 47 ; et/ou
- qu'il entre d'autres informations le concernant, telles que son nom et son adresse par exemple, qui pourront figurer sur l'ordonnance.

A l'étape d'entrée de données E1, dans un mode de réalisation préféré, il est également demandé au patient, via le système de génération d'instructions 12, de réaliser une mesure à l'aide du réfractomètre automatique 27 pour mesurer la réfraction oculaire objective RO de ses yeux.

En variante, notamment en cas de dispositif 1 dépourvu de réfractomètre automatique 27, on peut également demander du patient de saisir (via l'unité de saisie de données 11) la réfraction oculaire objective de ses yeux s'il la connaît.

A l'étape d'entrée de données E1, dans un mode de réalisation préféré mais non exclusif, il est également demandé au patient, via le système de génération d'instructions 12, de poser ses lunettes dans le frontofocomètre 29 pour que ce dernier mesure automatiquement les corrections des verres de ses lunettes.

L'étape de génération E2 suivante, mise en œuvre par l'unité centrale 9, consiste à générer automatiquement au moins un test oculaire, à l'aide notamment de l'âge du patient, de sa réfraction oculaire objective RO et de sa correction optique portée mesurée à l'aide d'un frontofocomètre 29.

Le ou les tests oculaires sont déterminés par l'unité centrale 9 pour présenter, à partir de la réfraction oculaire objective RO, des combinaisons de verres correcteurs différents au patient. Le patient regarde des affichages commandés par l'unité centrale 9 au travers du dispositif oculaire 3 pourvu de ces verres correcteurs, et il fournit des indications quant à sa vision par l'intermédiaire du système d'interface 14 à l'étape de test E3.

En fonction de ces indications du patient, l'unité centrale 9 détermine ensuite une réfraction RB (ou réfraction de brouillard), puis en déduit la réfraction oculaire subjective RF, à l'étape de traitement E4.

Dans un mode de réalisation préféré, l'étape de génération E2 utilise un test de brouillard. Bien entendu, d'autres types de tests peuvent être mis en œuvre par le dispositif 1, dans le cadre de la présente invention.

De préférence, le test de brouillard consiste à réaliser des affichages (regardés par le patient au travers des verres correcteurs présentés dans le dispositif oculaire 3) de lettres E, avec des branches des E affichées dans quatre orientations différentes, à savoir vers la droite, vers le bas, vers la gauche et vers le haut. On prévoit l'affichage de quatre E d'orientations différentes, avec dans le dispositif oculaire 3 la correction RO du patient et des E de 2/10. Le patient regarde ces affichages à travers les verres correcteurs agencés dans le dispositif oculaire 3. La vision est éclaircie progressivement (et rendue possible) en retirant progressivement du brouillard.

De préférence, avec la technique du brouillard, le dispositif 1 effectue le test sur un seul œil pour déterminer la réfraction RB. Par convention, on utilise l'œil droit, sauf si la mesure avec le réfractomètre automatique 27 est impossible sur l'œil droit.

A l'étape de test E3, pour guider le patient, le système de génération d'instructions 12 peut émettre (à la sous-étape E3A) le message vocal suivant destiné au patient : « le test de lecture suivant est appelé test du brouillard. Il est utilisé pour déterminer précisément votre correction. Votre vision va être volontairement brouillée au travers des lunettes, puis peu à peu s'éclaircira. Nous allons afficher des lettres E avec des branches dans quatre orientations différentes comme suit. Vers la droite, vers le bas, vers la gauche et vers le haut. Au départ, il vous sera totalement impossible de répondre puis, peu à peu, nous éclaircirons votre vision en retirant du brouillard. Le plus important pour ce test est de répondre uniquement lorsque vous devinez bien l'orientation du E. Lorsque vous y arriverez, indiquez-nous la direction du E à l'aide de la manette directionnelle, à savoir haut, bas, droite, gauche ». Cette manette directionnelle correspond à la manette 25 décrite ci-dessus.

A titre d'illustration, la méthode de calcul de la réfraction RB est la suivante, la réfraction RB étant obtenue en partant de la réfraction RO à laquelle on ajoute + 4 dioptries.

On affiche les quatre E possibles, puis on intègre dans le dispositif oculaire 3 du réfracteur automatique 2 des verres correcteurs de RO + 4 de dioptrie. On affiche un seul E de taille 2/10, et on change d'orientation après chaque réponse. Dans ce cas :

- s'il apparaît une erreur dans la réponse du patient ou si le patient ne peut pas répondre, on retire 0,50 de brouillard jusqu'à avoir retiré une dioptrie de brouillard, puis on retire 0,25 par pas ;
- si quatre réponses consécutives sont justes, on arrête le test ;
- de même, si on a retiré 2,75, on arrête le test.

L'unité centrale 9 détermine la réfraction RB à l'aide de l'expression suivante : RB = réfraction permettant de lire 2/10 - 1,25 (dioptries de sphère).

Une fois la réfraction RB déterminée pour un œil, l'unité centrale 9 peut soit déterminer la réfraction RB pour l'autre œil de la même façon, soit mettre en œuvre les calculs suivants :

- déterminer une valeur d'accommodation Δ, à l'aide de l'expression suivante :

$$\Delta = RB - RO$$

sur l'œil testé ; et

- retirer cette valeur d'accommodation Δ sur l'autre œil, sans l'avoir testé.

Finalement, l'étape de traitement E4, mise en œuvre par l'unité centrale 9, consiste notamment à déterminer automatiquement la réfraction oculaire subjective RF.

L'unité centrale 9 calcule la réfraction oculaire subjective RF, à partir de la réfraction RB déterminée de la manière décrite ci-dessus.

Pour ce faire, l'unité centrale 9 prend en compte la valeur d'accommodation Δ (telle que Δ = RB - RO ) du patient, de la manière suivante, pour déterminer la réfraction oculaire subjective RF :

- si

$$\Delta = 0, \ RF = RO = RB \ ;$$

- si

$$\Delta < 1{,}00 \ \text{dioptrie}, \ RF = RO + 0{,}25 \ ;$$

- si $\Delta \geq 1{,}00$ dioptrie, alors :

    • si RB est de sphère positive, la valeur RF est déterminée à partir de la valeur RB, en tenant compte d'un tableau de correspondance prédéterminé entre les valeurs RB et RF ;
    • si RB est de sphère négative, on réalise un test « rouge vert » pour déterminer la réfraction oculaire subjective RF.

L'étape de test E4 consiste également à générer une ordonnance P, à partir de cette réfraction oculaire subjective RF. Plus précisément, l'unité centrale 9 détermine la prescription P qui sera inscrite sur l'ordonnance, de la manière suivante :

- si $\Delta = 0$ ou si $\Delta < 1{,}00$ dioptrie ou bien si le patient a plus de 38 ans :

    • si RF est de sphère négative, alors P = RF telle que validée ;
    • si RF est de sphère positive, alors P = RF obtenue à partir du tableau de correspondance ;

- si $\Delta \geq 1{,}00$ dioptrie : P = RF.

Finalement, l'ordonnance P est fournie au patient, par exemple en étant imprimée par l'élément d'impression 19. L'ordonnance P peut également être transmise à distance à un tiers, par exemple à un professionnel de la santé, à l'aide de l'élément de transmission 21.

On peut également prévoir que le dispositif 1 émette un message d'alerte conseillant au patient de consulter un professionnel de la vision en urgence, si son acuité visuelle est trop faible.

Le procédé décrit ci-dessus est donc un procédé de détermination automatisé de la réfraction oculaire subjective d'un patient, à l'aide d'un algorithme (intégré dans l'unité centrale 9) et d'une analyse par intelligence artificielle de la combinaison de données de réfraction objective mesurées automatiquement à l'aide notamment d'un réfractomètre automatique 27, de l'âge du patient, de sa correction optique portée mesurée à l'aide d'un frontofocomètre 29 et de ses réponses recueillies par l'unité centrale 9, les différents éléments du dispositif 1 étant pilotés automatiquement par l'unité centrale 9, l'unité centrale 9 présentant dans les oculaires 4A et 4B du dispositif oculaire 3 une série de verres correcteurs adaptés, le ou les tests oculaires étant mis en œuvre avec un guidage vocal du patient par une voix synthétisée (par l'intermédiaire du système de génération d'instructions 12) et comprenant un recueil de réponses à l'aide du système d'interface 14.

## Revendications

1. Dispositif de détermination automatique au moins de la réfraction oculaire subjective d'un patient, ledit dispositif (1) comportant :

   - un réfracteur automatique (2) comprenant un dispositif oculaire (3) et une unité de gestion (5) configurée pour prélever des verres correcteurs d'une réserve (6) et les positionner dans le dispositif oculaire (3) ;
   - une unité centrale (9) configurée pour au moins commander l'unité de gestion (5) du réfracteur automatique (2) afin de positionner des verres correcteurs dans le dispositif oculaire (3) ;
   - une unité de saisie de données (11) permettant d'entrer dans l'unité centrale (9) au moins des données relatives au patient ;
   - un système de génération d'instructions (12) commandé par l'unité centrale (9) et configuré pour générer automatiquement des instructions destinées au patient au moins lors d'un test oculaire ;
   - un système d'interface (14) entre le patient et l'unité centrale (9), qui est configuré pour détecter, directement, des indications générées sous forme gestuelle par le patient au moins lors dudit test oculaire et pour transmettre directement ces instructions à l'unité centrale (9) ;
   - un réfractomètre (27) automatique configuré pour mesurer la réfraction oculaire objective du patient et pour la transmettre à l'unité centrale (9), la réfraction oculaire objective mesurée étant utilisée par l'unité centrale (9) pour générer ledit au moins un test oculaire et pour déterminer ladite réfraction oculaire subjective ; et
   - un frontofocomètre (29) configuré pour mesurer les corrections de verres de lunettes du patient et pour transmettre les corrections mesurées à l'unité centrale (9), les corrections mesurées étant utilisées par l'unité centrale (9) pour générer ledit au moins un test oculaire et pour déterminer ladite réfraction oculaire subjective, l'unité centrale (9) pilotant automatiquement les éléments du dispositif (1) et étant configurée également pour mettre en œuvre automatiquement ledit au moins un test oculaire, en commandant l'unité de gestion (5) et en recevant les indications générées par le patient par l'intermédiaire du système d'interface (14), et pour déterminer automatiquement la réfraction oculaire subjective à partir au moins desdites indications reçues ainsi qu'une prescription de verres correcteurs, adaptée à ladite réfraction oculaire subjective ainsi déterminée.

2. Dispositif selon la revendication 1, **caractérisé en ce que** l'unité centrale (9) est configurée pour générer une ordonnance comprenant une prescription de lunettes pour le patient, adaptée à ladite réfraction oculaire subjective.

3. Dispositif selon la revendication 2, **caractérisé en ce qu'**il comporte, de plus, au moins l'un des éléments suivants :

   - un élément d'enregistrement (18) configuré pour enregistrer l'ordonnance ;
   - un élément d'impression (19) configuré pour imprimer l'ordonnance ;
   - un élément de transmission (21) configuré pour transmettre l'ordonnance à distance à un utilisateur.

4. Dispositif selon l'une quelconque des revendications précédentes, **caractérisé en ce que** le système de génération d'instructions (12) comporte une unité d'émission vocale.

5. Dispositif selon l'une quelconque des revendications précédentes, **caractérisé en ce que** le système d'interface (14) comporte au moins un organe d'actionnement manuel (23).

6. Dispositif selon l'une quelconque des revendications précédentes, **caractérisé en ce que** le système d'interface (14) comporte au moins un système (26) de détection automatique de gestes du patient.

7. Dispositif selon l'une quelconque des revendications 1 à 6, **caractérisé en ce qu'**il comporte un réseau local (32) configuré pour permettre des communications entre au moins certains des éléments du dispositif (1).

8. Dispositif selon l'une quelconque des revendications 1 à 6, **caractérisé en ce qu'**au moins certains des éléments du dispositif (1) communiquent entre eux par l'intermédiaire de liaisons série (41).

9. Cabine de détermination automatique de la réfraction oculaire subjective d'un patient, **caractérisée en ce qu'**elle est au moins partiellement fermée et **en ce qu'**elle comporte au moins un siège (44) pour un patient et un dispositif (1) de détermination automatique de la réfraction oculaire subjective d'un patient, selon l'une quelconque des revendications 1 à 8.

10. Cabine selon la revendication 9, **caractérisée en ce que** les différents éléments dudit dispositif (1) de détermination automatique de la réfraction oculaire subjective d'un patient, sont agencés de manière à être accessibles, si nécessaire, à partir du siège (44) du patient.

11. Procédé de détermination automatique de la réfraction oculaire subjective d'un patient, à l'aide d'un dispositif (1) comportant au moins :

   - un réfracteur automatique (2) comprenant un dispositif oculaire (3) et une unité de gestion (5) configurée pour prélever des verres correcteurs d'une réserve (6) et les positionner dans le dispositif oculaire (3) ;
   - une unité centrale (9) ;
   - une unité de saisie de données (11) ;
   - un système de génération d'instructions (12) ;
   - un système d'interface (14) entre le patient et l'unité centrale (9),
   - un réfractomètre (27) automatique ; et
   - un frontofocomètre (29), ledit procédé comportant:
   - une étape d'entrée de données (E1) consistant à entrer des données dans l'unité centrale (9) qui pilote automatiquement les éléments du dispositif (1), le réfractomètre (27) automatique mesurant la réfraction oculaire objective du patient et transmettant la réfraction oculaire objective mesurée à l'unité centrale (9), le frontofocomètre (29) mesurant les corrections de verres de lunettes du patient et transmettant les corrections mesurées à l'unité centrale (9), la réfraction oculaire objective mesurée et les corrections mesurées étant utilisées par l'unité centrale (9) pour générer ledit au moins un test oculaire, déterminer ladite réfraction oculaire subjective ;
   - une étape de génération (E2), mise en œuvre par l'unité centrale (9), et consistant à générer automatiquement au moins un test oculaire, à l'aide au moins de données entrées, relatives au patient ;
   - une étape de test (E3), consistant à mettre en œuvre ledit au moins un test oculaire généré à l'étape de génération (E2), l'étape de test (E3) comprenant au moins :

      • une sous-étape (E3A) de génération d'instructions pour le patient, mise en œuvre par le système de génération d'instructions (12) ;
      • une sous-étape (E3B) de commande de l'unité de gestion (5) du réfracteur automatique (2) pour prélever des verres correcteurs de la réserve (6) et les positionner dans le dispositif oculaire (3) ;
      • une sous-étape (E3C) de détection directe d'indications générées sous forme gestuelle par le patient au cours du test oculaire, en regardant des affichages à travers le dispositif oculaire (3), et de transmission directe de ces indications à l'unité centrale (9), mise en œuvre par le système d'interface (14) ; et

   - une étape de traitement (E4), mise en œuvre par l'unité centrale (9), et consistant à déterminer automatiquement, à partir au moins desdites indications reçues, la réfraction oculaire subjective du patient ainsi qu'une prescription de verres correcteurs, adaptée à ladite réfraction oculaire subjective ainsi déterminée.

12. Procédé selon la revendications 11, **caractérisé en ce que** l'étape de génération (E2) et l'étape de test (E3) utilisent un test de brouillard.

13. Procédé selon l'une des revendications 11 et 12, **caractérisé en ce que** l'étape de test (E3) consiste à afficher des lettres E avec des branches présentant des orientations différentes.

**Patentansprüche**

1. Vorrichtung zur automatischen Bestimmung mindestens der subjektiven Augenrefraktion eines Patienten, wobei die Vorrichtung (1) umfasst:

   - einen Autorefraktor (2), der eine Okularvorrichtung (3) und eine Verwaltungseinheit (5) umfasst, die so konfiguriert ist, dass sie Korrektionsgläser aus einem Lager (6) entnimmt und sie in der Okularvorrichtung (3) positioniert;
   - eine Zentraleinheit (9), die so konfiguriert ist, dass sie mindestens die Verwaltungseinheit (5) des Autorefraktors (2) steuert, um Korrektionsgläser in der Okularvorrichtung (3) zu positionieren;

- eine Datenerfassungseinheit (11), die es ermöglicht, mindestens patientenbezogene Daten in die Zentraleinheit (9) einzugeben;

- ein Anweisungserzeugungssystem (12), das von der Zentraleinheit (9) gesteuert wird und so konfiguriert ist, dass es mindestens bei einem Sehtest automatisch Anweisungen erzeugt, die für den Patienten bestimmt sind;

- ein Schnittstellensystem (14) zwischen dem Patienten und der Zentraleinheit (9), das so konfiguriert ist, dass es Angaben, die vom Patienten mindestens beim Sehtest in Gestenform erzeugt werden, direkt erkennt und diese Anweisungen direkt an die Zentraleinheit (9) überträgt;

- ein Autorefraktometer (27), das so konfiguriert ist, dass es die objektive Augenrefraktion des Patienten misst und diese an die Zentraleinheit (9) überträgt, wobei die gemessene objektive Augenrefraktion von der Zentraleinheit (9) verwendet wird, um den mindestens einen Sehtest zu erzeugen und die subjektive Augenrefraktion zu bestimmen; und

- ein Frontofokometer (29), das so konfiguriert ist, dass es die Brillenglaskorrektionen des Patienten misst und die gemessenen Korrektionen an die Zentraleinheit (9) überträgt, wobei die gemessenen Korrektionen von der Zentraleinheit (9) verwendet werden, um den mindestens einen Sehtest zu erzeugen und die subjektive Augenrefraktion zu bestimmen,

wobei die Zentraleinheit (9) die Elemente der Vorrichtung (1) automatisch steuert und auch so konfiguriert ist, dass sie den mindestens einen Sehtest automatisch umsetzt, indem sie die Verwaltungseinheit (5) steuert und die vom Patienten erzeugten Angaben mittels des Schnittstellensystems (14) empfängt, und die subjektive Augenrefraktion mindestens auf Grundlage der empfangenen Angaben sowie eine Verordnung von Korrektionsgläsern, die für die so bestimmte subjektive Augenrefraktion geeignet ist, automatisch bestimmt.

2. Vorrichtung nach Anspruch 1, **dadurch gekennzeichnet, dass** die Zentraleinheit (9) so konfiguriert ist, dass sie ein Rezept erzeugt, das eine Brillenverordnung für den Patienten umfasst, die für die subjektive Augenrefraktion geeignet ist.

3. Vorrichtung nach Anspruch 2, **dadurch gekennzeichnet, dass** sie zusätzlich mindestens eines der folgenden Elemente umfasst:

- ein Speicherelement (18), das so konfiguriert ist, dass es das Rezept speichert;
- ein Druckelement (19), das so konfiguriert ist, dass es das Rezept druckt;
- ein Übertragungselement (21), das so konfiguriert ist, dass es das Rezept aus der Ferne an einen Benutzer überträgt.

4. Vorrichtung nach einem der vorstehenden Ansprüche, **dadurch gekennzeichnet, dass** das Anweisungserzeugungssystem (12) eine Sprachausgabeeinheit umfasst.

5. Vorrichtung nach einem der vorstehenden Ansprüche, **dadurch gekennzeichnet, dass** das Schnittstellensystem (14) mindestens ein manuelles Bedienglied (23) umfasst.

6. Vorrichtung nach einem der vorstehenden Ansprüche, **dadurch gekennzeichnet, dass** das Schnittstellensystem (14) mindestens ein System (26) zur automatischen Erkennung von Gesten des Patienten umfasst.

7. Vorrichtung nach einem der Ansprüche 1 bis 6, **dadurch gekennzeichnet, dass** sie ein lokales Netzwerk (32) umfasst, das so konfiguriert ist, dass es Kommunikationen zwischen mindestens einigen der Elemente der Vorrichtung (1) ermöglicht.

8. Vorrichtung nach einem der Ansprüche 1 bis 6, **dadurch gekennzeichnet, dass** mindestens einige der Elemente der Vorrichtung (1) mittels serieller Verbindungen (41) untereinander kommunizieren.

9. Kabine zur automatischen Bestimmung der subjektiven Augenrefraktion eines Patienten, **dadurch gekennzeichnet, dass** sie mindestens teilweise geschlossen ist und dadurch, dass sie mindestens einen Sitz (44) für einen Patienten und eine Vorrichtung (1) zur automatischen Bestimmung der subjektiven Augenrefraktion eines Patienten nach einem der Ansprüche 1 bis 8 umfasst.

10. Kabine nach Anspruch 9, **dadurch gekennzeichnet, dass** die verschiedenen Elemente der Vorrichtung (1) zur automatischen Bestimmung der subjektiven Augenrefraktion eines Patienten so angeordnet sind, dass sie, falls erforderlich, vom Sitz (44) des Patienten aus zugänglich sind.

11. Verfahren zur automatischen Bestimmung der subjektiven Augenrefraktion eines Patienten mithilfe einer Vorrichtung (1), die mindestens umfasst:

- einen Autorefraktor (2), der eine Okularvorrichtung (3) und eine Verwaltungseinheit (5) umfasst, die so konfiguriert ist, dass sie Korrektionsgläser aus einem Lager (6) entnimmt und sie in der Okularvorrichtung (3) positioniert;
- eine Zentraleinheit (9);
- eine Datenerfassungseinheit (11);
- ein Anweisungserzeugungssystem (12);
- ein Schnittstellensystem (14) zwischen dem Patienten und der Zentraleinheit (9),
- ein Autorefraktometer (27); und
- ein Frontofokometer (29),

wobei das Verfahren umfasst:

- einen Dateneingabeschritt (E1), der darin besteht, Daten in die Zentraleinheit (9) einzugeben, die die Elemente der Vorrichtung (1) automatisch steuert, wobei das Autorefraktometer (27) die objektive Augenrefraktion des Patienten misst und die gemessene objektive Augenrefraktion an die Zentraleinheit (9) überträgt, wobei das Frontofokometer (29) die Brillenglaskorrektionen des Patienten misst und die gemessenen Korrektionen an die Zentraleinheit (9) überträgt, wobei die gemessene objektive Augenrefraktion und die gemessenen Korrektionen von der Zentraleinheit (9) verwendet werden, um den mindestens einen Sehtest zu erzeugen, die subjektive Augenrefraktion zu bestimmen;
- einen Erzeugungsschritt (E2), der von der Zentraleinheit (9) umgesetzt wird und darin besteht, automatisch mindestens einen Sehtest mithilfe mindestens von eingegebenen Daten, die sich auf den Patienten beziehen, zu erzeugen;
- einen Testschritt (E3), der darin besteht, den mindestens einen im Erzeugungsschritt (E2) erzeugten Sehtest umzusetzen, wobei der Testschritt (E3) mindestens umfasst:
- einen Teilschritt (E3A) des Erzeugens von Anweisungen für den Patienten, der vom Anweisungserzeugungssystem (12) umgesetzt wird;
- einen Teilschritt (E3B) des Steuerns der Verwaltungseinheit (5) des Autorefraktors (2), um Korrektionsgläser aus dem Lager (6) zu entnehmen und sie in der Okularvorrichtung (3) zu positionieren;
- einen Teilschritt (E3C) des direkten Erkennens von Angaben, die vom Patienten während des Sehtests in Gestenform erzeugt werden, indem er Anzeigen durch die Okularvorrichtung (3) betrachtet, und zur direkten Übertragung dieser Angaben an die Zentraleinheit (9), der vom Schnittstellensystem (14) umgesetzt wird; und
- einen Verarbeitungsschritt (E4), der von der Zentraleinheit (9) umgesetzt wird und darin besteht, automatisch, mindestens auf Grundlage der empfangenen Angaben, die subjektive Augenrefraktion des Patienten sowie eine Verordnung von Korrektionsgläsern, die für die so bestimmte subjektive Augenrefraktion geeignet ist, zu bestimmen.

12. Verfahren nach Anspruch 11,
**dadurch gekennzeichnet, dass** der Erzeugungsschritt (E2) und der Testschritt (E3) einen Nebeltest verwenden.

13. Verfahren nach einem der Ansprüche 11 und 12, **dadurch gekennzeichnet, dass** der Testschritt (E3) darin besteht, Buchstaben E mit Armen anzuzeigen, die unterschiedliche Ausrichtungen aufweisen.

## Claims

1. Device for automatic determination of at least the subjective ocular refraction of a patient, said device (1) comprising:

- an automatic refractor (2) comprising an ocular device (3) and a management unit (5) configured to take corrective lenses from a reserve (6) and position them in the ocular device (3);
- a central unit (9) configured to at least control the management unit (5) of the automatic refractor (2) in order to position corrective lenses in the ocular device (3);
- a data input unit (11) for inputting at least patient data into the central unit (9),
- an instruction generation system (12) controlled by the central unit (9) and configured to automatically generate instructions to the patient at least during an eye test;
- an interface system (14) between the patient and the central unit (9), which is configured to directly detect indications generated in the form of gestures by the patient at least during said eye test and to transmit these instructions directly to the central unit (9);
- an automatic refractometer (27) configured to measure the objective ocular refraction of the patient and to transmit it to the central unit (9), the measured objective ocular refraction being used by the central unit (9) to generate said at least one eye test and to determine said subjective ocular refraction; and
- a frontofocometer (29) configured to measure the corrections of the patient's eyeglass lenses and to transmit the measured corrections to the central unit (9), the measured corrections being used by the central unit (9) to generate said at least one eye test and to determine said subjective ocular refraction,

the central unit (9) automatically controlling the elements of the device 1 and being also configured to automatically implement said at least one eye test, by controlling the management unit (5) and receiving the indications generated by the patient via the interface system (14), and to automatically determine the subjective ocular refraction from at least said received indications and a prescription for corrective lenses, adapted to the subjective ocular refraction thus determined.

2. Device according to claim 1,
   **characterised in that** the central unit (9) is configured to generate a prescription comprising a prescription for spectacles for the patient, adapted to said subjective ocular refraction.

3. Device according to claim 2,
   **characterised in that** it further comprises at least one of the following elements:

   - a recording element (18) configured to record the prescription;
   - a print element (19) configured to print the prescription;
   - a transmission element (21) configured to transmit the prescription remotely to a user.

4. Device according to any one of the preceding claims, **characterised in that** the instruction-generating system (12) comprises a voice transmission unit.

5. Device according to any one of the preceding claims, **characterised in that** the interface system (14) comprises at least one manual actuating element (23).

6. Device according to any one of the preceding claims, **characterised in that** the interface system (14) comprises a system (26) for automatic detection of patient gestures.

7. Device according to any one of claims 1 to 6,
   **characterised in that** it comprises a local area network (32) configured to allow communications between at least some of the elements of the device (1).

8. Device according to any one of claims 1 to 6,
   **characterised in that** at least some of the elements of the device (1) communicate with each other via serial links (41).

9. Booth for the automatic determination of the subjective ocular refraction of a patient,
   **characterised in that** it is at least partially closed and **in that** it comprises at least one seat (44) for a patient and a device (1) for the automatic determination of the subjective ocular refraction of a patient, according to any one of claims 1 to 8.

10. Booth according to claim 9,
    **characterised in that** the various elements of said device (1) for automatic determination of the subjective ocular refraction of a patient are arranged so as to be accessible, if necessary, from the patient's seat (44).

11. Method for the automatic determination of the subjective ocular refraction of a patient, using a device (1) comprising at least:

    - an automatic refractor (2) comprising an ocular device (3) and a management unit (5) configured to take corrective lenses from a reserve (6) and position them in the ocular device (3);
    - a central unit (9);
    - a data input unit (11);
    - an instruction-generating system (12);
    - an interface system (14) between the patient and the central unit (9);
    - an automatic refractometer (27); and
    - a frontofocometer (29),

    said method comprising:

    - a data input step (E1) consisting of inputting data into the central unit (9) which automatically controls the elements of the device 1, the automatic refractometer (27) measuring the objective ocular refraction of the patient and transmitting the measured objective ocular refraction to the central unit (9), the frontofocometer (29) measuring the corrections of the patient's eyeglass lenses and transmitting the measured corrections to the central unit (9), the measured objective ocular refraction and the measured corrections being used by the central unit (9) to generate said at least one eye test, to determine said subjective ocular refraction;
    - a generation step (E2), implemented by the central unit (9), and consisting of automatic generating at least one eye test, using at least inputted data relating to the patient;
    - a test step (E3), consisting of implementing at least one eye test generated in the generation step (E2), the test step (E3) comprising at least:

      • a sub-step (E3A) of generation of instructions for the patient, implemented by the instruction-generating system (12);
      • a sub-step (E3B) of directing the management unit (5) of the automatic refractor (2) to take corrective lenses from the reserve (6) and position them in the ocular device (3);

• a sub-step (E3C) of direct detection of indications generated in the form of gestures by the patient during the eye test, by viewing displays through the ocular device (3), and of direct transmission of these indications to the central unit (9), implemented by the interface system (14); and

- a processing step (E4), implemented by the central unit (9), and consisting of automatic determining the subjective ocular refraction of the patient and a prescription for corrective lenses, adapted to the subjective ocular refraction thus determined, from at least said indications received.

**12.** Method according to claim 11, **characterised in that** the generation step (E2) and the test step (E3) use a fog test.

**13.** Method according to one of claims 11 and 12, **characterised in that** the test step (E3) consists of displaying letter Es with branches having different orientations.

Fig. 1

Fig. 2

Fig. 3

Fig. 4

Fig. 5

Fig. 6

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3050922
- US 2009153796 A
- US 2015070650 A